# EUROPÄISCHE PATENTSCHRIFT

(11) **EP 2 651 515 B1**
(45) Veröffentlichungstag und Bekanntmachung des Hinweises auf die Patenterteilung: **02.01.2019**
(21) Anmeldenummer: 11788421.3
(22) Anmeldetag: 21.11.2011
(51) Int. Cl.: A61Q 5/04, A61Q 5/10, A61K 8/898

(54) **MITTEL ZUR FARB- UND/ODER FORMVERÄNDERUNG KERATINISCHER FASERN**
AGENT FOR CHANGING THE COLOUR AND/OR FORM OF KERATINOUS FIBRES
AGENTS POUR LA MODIFICATION DE COULEUR ET/OU DE FORME DE FIBRES KÉRATINIQUES

(30) Priorität: 16.12.2010 DE 102010063210
(43) Veröffentlichungstag der Anmeldung: 23.10.2013
(73) Patentinhaber: Henkel AG & Co. KGaA, 40589 Düsseldorf (DE)
(72) Erfinder: GOUTSIS, Konstantin, 41812 Erkelenz (DE); JANßEN, Frank, 41470 Neuss (DE)
(86) Internationale Anmeldenummer: PCT/EP2011/070542
(87) Internationale Veröffentlichungsnummer: WO 2012/079915

(56) Entgegenhaltungen:
- WO-A2-2011/139265
- WO-A2-2012/024364
- WO-A2-2012/027369
- US-A- 5 143 518
- US-A1- 2004 133 996
- US-A1- 2009 000 638
- MOMENTIVE: "Silsoft AX", INTERNET CITATION, 1. März 2010 (2010-03-01), Seiten 1-12, XP002675429, Gefunden im Internet: URL:http://www.silicones.com/momentiveInte rnetDoc/Internet/Static%20Files/Documents/ Marketing%20Bulletin/Silsoft%20AX%20MB%20i ndd.pdf [gefunden am 2012-05-08]
- MOMENTIVE: "Momentive introduces Silsoft AX conditioning agent and Tospearl AQ microspheres; advanced personal care materials", INTERNET CITATION, 10. März 2010 (2010-03-10), Seite 1, XP002675427, Gefunden im Internet: URL:http://www.silicones.com/momentiveinte rnetdoc/internet/static%20files/press%20do cuments/2010/silsoft_ax_tospearl_aq_releas e_final_031010.pdf [gefunden am 2012-05-07]

## Beschreibung

Die Erfindung liegt auf dem Gebiet der Kosmetik und betrifft Mittel zum Färben keratinischer Fasern, die mindestens eine spezifische Silikonverbindung enthalten.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden.
Dabei können Dauerwell- und andere die Haarform verändernde Verfahren nahezu unabhängig vom Typ der zu behandelnden Haare eingesetzt werden. Gleichzeitig oder alternativ zu der Formveränderung besteht aber auch vielfach der Wunsch, die Haarfarbe zu ändern und zusammen mit der Haarfärbung auch eine Aufhellung des zu färbenden Haares zu erreichen.

Diese hierzu verwendeten Mittel sollen neben der gewünschten Färbe- und Formleistung möglichst minimale Schädigungen auf dem Haar hervorrufen.
Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.
Für permanente, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente so genannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese dann im Rahmen oxidativer Prozesse im Haar naturanaloge Farbstoffe ausbilden.
Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Oxidative Färbemittel enthalten in der Regel Wasserstoffperoxid als Oxidationsmittel. Dies führt neben dem gewünschten kosmetischen Effekt zu oxidativen Schädigungen der Haaroberfläche. Auch führt der üblicherweise in solchen Mitteln basisch eingestellte pH-Wert dazu, dass die Struktur des Haares oxidativ geschädigt wird. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids ist, desto stärkere Schädigungen werden in der Regel auf der Keratinfaser hervorgerufen. Insbesondere Spliss oder gar Bruch der Faser sind vom Verbraucher höchst unerwünschte Begleiterscheinungen einer oxidativen Haarbehandlung, die insbesondere bei mehrfacher Wiederholung der oxidativen Haarbehandlung auftreten können. Es ist daher besonders wünschenswert, Färbemittel bereitzustellen, die neben einer guten Färbeleistung einen positiven Beitrag zur Verbesserung der Faserstruktur leisten und damit gleichzeitig als Pflege- oder Konditioniermittel wirken. Somit könnte auf die sonst häufig erforderliche, zusätzliche pflegende Nachbehandlung verzichtet werden, die sowohl hinsichtlich Anwendungskomforts als zusätzlichen Behandlungsschritt als auch hinsichtlich Verpackungsökonomie mit Nachteilen behaftet ist.

Die dauerhafte Verformung von Keratinfasern wird üblicherweise so durchgeführt, dass man die Faser mechanisch verformt und die Verformung durch geeignete Hilfsmittel festlegt. Vor und/oder nach dieser Verformung behandelt man die Faser mit der wässrigen Zubereitung einer keratinreduzierenden Substanz und spült nach einer Einwirkungszeit mit Wasser oder einer wässrigen Lösung. In einem zweiten Schritt behandelt man dann die Faser mit der wässrigen Zubereitung eines Oxidationsmittels. Nach einer Einwirkungszeit wird auch dieses ausgespült und die Faser von den mechanischen Verformungshilfsmitteln (Wickler, Papilloten) befreit.
Die wässrige Zubereitung der keratinreduzierenden Substanz ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so dass es zu einer Lockerung der Peptidvernetzung und infolge der Spannung der Faser durch die mechanische Verformung zu einer Neuorientierung des Keratingefüges kommt. Unter dem Einfluss des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.
Auch bei der Dauerwellbehandlung werden die Fasern aggressiven Medien ausgesetzt, so dass ein Bedürfnis besteht, die Mittel hinsichtlich ihrer pflegenden Eigenschaften weiter zu verbessern.

Die im Rahmen dieser Methoden einsetzbaren Wirkstoffe müssen insbesondere hinsichtlich ihrer Stabilität hohen Ansprüchen genügen, da die Färbecremes und Wellmittel üblicherweise einen hohen pH-Wert und die Oxidationsmittelzubereitungen einen niedrigen pH-Wert aufweisen. Auch müssen diese Wirkstoffe gegenüber den reduzierenden Bedingungen der Wellmittel stabil sein. Ferner sind Unverträglichkeiten der verschiedenen Wirkstoffe untereinander und somit eine geringe Lagerstabilität zu vermeiden.

US 2004/0133996 A1 offenbart Mittel zum Färben von Haaren, die neben Farbstoffen auch Polymere und Silikonöle bzw. Silikongums enthalten.

Es besteht daher weiterhin Bedarf an Wirkstoffen, die unter den Bedingungen der oxidativen Farbveränderung sowie der Dauerwellbehandlung keratinischer Fasern einen positiven Einfluss auf den Pflegezustand der Haare ausüben. Ferner besteht ein Interesse an Wirkstoffen, die weiterhin besonders gut die primären Ziele der erfindungsgemäßen Mittel unterstützen und zu intensiven Färbungen, guten oder besseren Echtheitseigenschaften und/oder zu stabileren Dauerwellen führen.

Aufgabe der vorliegenden Erfindung ist es daher, die oben genannten Nachteile von handelsüblichen Mitteln zur Veränderung der Haarfarbe und/oder -form herabzusenken und Mittel mit guten, vorzugsweise verbesserten Farb- und/ oder Formveränderungseigenschaften und guten pflegenden Eigenschaften, insbesondere im Hinblick auf verbesserte Kämmbarkeit der Haare, bereit zu stellen. Es wurde nun gefunden, dass Mittel zur Farbveränderung keratinischer Fasern, die neben mindestens einer farbverändernden Verbindung weiterhin mindestens eine spezifische Silikonverbindung enthalten, diese positiven Eigenschaften aufweisen.

Erster Gegenstand der vorliegenden Anmeldung sind daher Mittel zur Farbveränderung keratinischer Fasern, enthaltend in einem kosmetisch akzeptablen Träger
a. mindestens eine farbgebende Verbindung ausgewählt aus mindestens einem Oxidationsfarbstoffvorprodukt
dadurch gekennzeichnet, dass das Mittel weiterhin
a. mindestens eine Verbindung der Formel (I) enthält. 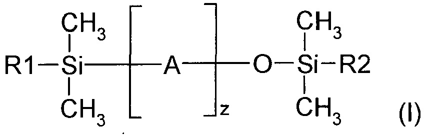 mit 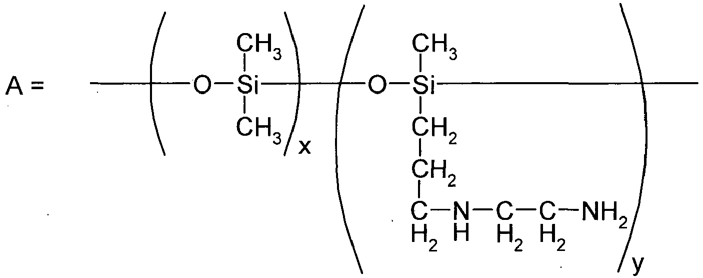 worin
   i. x und y unabhängig von einander für Werte zwischen 1 und 100 stehen und
   ii. z für Werte zwischen 1 und 100 steht, wobei, falls z ≥ 2, die jeweiligen Werte x und y in einem Strukturelement A jeweils unabhängig von vorangehenden Strukturelementen A gewählt werden können und
   iii. R1 und R2 unabhängig voneinander für lineare C₁₄-C₂₀-Alkylketten stehen.

Die Strukturelemente A der Verbindung der Formel (I) sind erfindungsgemäß aus je einem oder mehreren Elementen 3-[(2-Aminoethyl)amino]propyl-methyl-siloxan und einem oder mehreren Elementen Dimethylsiloxan aufgebaut. Die Anzahl an Dimethylsiloxan Elementen wird durch den Parameter x definiert. Die Anzahl an 3-[(2-Aminoethyl)amino]propyl-methyl-siloxan Elementen wird durch den Parameter y definiert. Die Werte der Parameter x und y stehen erfindungsgemäß unabhängig voneinander für Zahlen zwischen 1 und 100.
Die Anzahl an Strukturelementen A wird durch den Parameter z vorgegeben. Erfindungsgemäß liegt der Wert des Parameters z zwischen 1 und 100. Falls z ≥ 2, können die Parameter x und y in jedem Strukturelement A unabhängig von vorangehenden Strukturelementen A gewählt werden. Daraus folgt, dass sich für den Fall z ≥ 2 die einzelnen Strukturelemente A in ihrer Anzahl an 3-[(2-Aminoethyl)amino]propyl-methyl-siloxan Elementen und/oder in ihrer Anzahl an Dimethylsiloxan Elementen voneinander unterscheiden können.

Das Siloxan-Grundgerüst der Verbindung der Formel (I) wird gemäß der vorliegenden Erfindung an beiden Enden von je einer Alkyldimethylsilyl-Gruppe terminiert.
Die Alkylreste R1 und R2 gemäß der Verbindung der Formel (I) stehen dabei unabhängig von einander für lineare Kohlenstoffketten, deren Kohlenstoffkette eine C₁₄-C₂₀ -Kette ist. Beispiele für erfindungsgemäße Fettalkylketten sind Tetradeyl, Pentadecyl, Hexadecyl, Heptadecyl, Octadecyl, Nonadecyl, Eicosyl. Insbesondere bevorzugt sind Hexadecyl (Cetyl) und/oder Octadecyl (Stearyl). Unter Cetearyl versteht sich eine Mischung aus Cetyl und Stearyl. Erfindungsgemäß besonders bevorzugt ist die Verbindung der Formel (I), in der die Reste R1 und R2 für Cetearyl stehen (Bis-Cetearyl Amodimethicon).

Erfindungsgemäß bevorzugt sind Mittel, die bezogen auf das Gesamtgewicht der anwendungsbereiten Mischung die Verbindung/en der Formel (I) in Mengen von 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-% enthalten.

Neben der/den Verbindung(en) der Formel (I) enthalten erfindungsgemäße Mittel mindestens eine farbgebende Verbindung ausgewählt aus mindestens einem Oxidationsfarbstoffvorprodukt.

Es sind solche Mittel erfindungsgemäß bevorzugt, die die Oxidationsfarbstoffvorprodukte bezogen auf das Gesamtgewicht des anwendungsbereiten Mittels von 0,001 bis 10 Gew.-%, bevorzugt, 0,01 bis 8 Gew.-%, besonders bevorzugt 0,1 bis 5 Gew.-% und insbesondere bevorzugt 0,5 bis 3 Gew.-% enthalten.

Als Oxidationsfarbstoffvorprodukte enthalten die Färbezubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 3 : 1 bis 1 : 3, insbesondere 2 : 1 bis 1 : 1, enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylehdiamin, 2,6-Dimethyl-p-phenylendiamin, N,N-Bis-(2-hydroxyethyl)-p-phenylendiamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methylphenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden ausgewählt N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan und Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol sowie deren physiologisch verträglichen Salze.

Ferner kann die Entwicklerkomponente ausgewählt sein aus o-Aminophenol und seinen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind die Verbindungen 2,4,5,6-Tetraaminopyrimidin, 4-Hydroxy-2,5,6-triaminopyrimidin und deren physiologisch verträglichen Salze. Bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind
(A) m-Aminophenol und dessen Derivate, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, wie 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}amino)ethanol,
(D) o-Diaminobenzol und dessen Derivate,
(E) Di-beziehungsweise Trihydroxybenzolderivate, insbesondere Resorcin, 2-Chlorresorcin, 4-Chlorresorcin, 2-Methylresorcin und 1,2,4-Trihydroxybenzol,
(F) Pyridinderivate, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxypyridin,
(G) Naphthalinderivate, wie1-Naphthol und 2-Methyl-1-naphthol,
(H) Morpholinderivate, wie 6-Hydroxybenzomorpholin,
(I) Chinoxalinderivate,
(J) Pyrazolderivate, wie 1-Phenyl-3-methylpyrazol-5-on,
(K) Indolderivate, wie 6-Hydroxyindol,
(L) Pyrimidinderivate oder
(M) Methylendioxybenzolderivate, wie 1-(2'-Hydroxyethyl)-amino-3,4-methylendioxybenzol
sowie deren physiologisch verträglichen Salze.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie eines deren physiologisch verträglichen Salze.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin;p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)-benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

In einer weiteren Ausführungsform der vorliegenden Erfindung enthalten die erfindungsgemäßen Mittel mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Besonders bevorzugte Derivate des Indolins sind das 5,6-Dihydroxyindolin und das 2,3-Dioxoindolin (Isatin) und deren physiologisch verträglichen Salze. Ein besonders bevorzugtes Derivat des Indols ist das 5,6-Dihydroxyindol und dessen physiologisch verträglichen Salze. Die erfindungsgemäßen Mittel enthalten die Indol- oder Indolin-Derivate vorzugsweise in einer Mengen von 0,05 bis 10 Gew.-%, bevorzugt 0,2 bis 5 Gew.-%, jeweils bezogen auf ihr Gesamtgewicht.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist.

Eine Ausführungsform des ersten Erfindungsgegenstandes sind daher Mittel, welche zusätzlich ein Oxidationsmittel, ausgewählt aus Wasserstoffperoxid sowie seinen festen Anlagerungsprodukten an organische und anorganische Verbindungen, enthalten. Als feste Anlagerungsprodukte kommen erfindungsgemäß insbesondere die Anlagerungsprodukte an Harnstoff, Melamin, Polyvinylpyrrolidinon sowie Natriumborat in Frage.

Das anwendungsbereite Mittel kann in einer besonderen Ausführungsform der Erfindung erst kurz vor der Anwendung aus einer Farbveränderungszubereitung und einer Oxidationsmittelzubereitung hergestellt werden.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung, bezogen auf ihr Gewicht, 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Weiterhin hat es sich als vorteilhaft erweisen, wenn die Mittel, insbesondere die Oxidationsmittelzubereitungen mindestens einen Stabilisator oder Komplexbildner enthalten. Im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta(methylen-phosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

In einer bevorzugten Ausführungsform des ersten Erfindungsgegenstands enthält das Mittel als farbverändernde Verbindung mindestens ein Aufhellmittel. Unter Aufhellmittel werden erfindungsgemäß Oxidationsmittel zum Aufhellen bzw. Blondieren der Haare verstanden.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel. In einer bevorzugten Ausführungsform enthält das Mittel als Oxidationsmittel bezogen auf sein Gewicht mindestens 1 Gew.-% Wasserstoffperoxid.

Soll helles oder aufgehelltes Haar bei der Behandlung eine Auffrischung des Aufhelleffekts erfahren, so kann es vorteilhaft sein, Wasserstoffperoxid und /oder eines seiner festen Anlagerungsprodukte in ein Shampoo oder ein ähnliches Behandlungsmittel einzuarbeiten. Damit wird jedoch in der Regel nur eine geringe Aufhellung erzielt. Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend.

In diesen Fällen wird in der Regel eine Kombination aus Wasserstoffperoxid und Aufhellmitteln eingesetzt, woraus eine Steigerung des Aufhellvermögens der Mittel resultiert. Bevorzugte Aufhellmittel sind Bleichkraftverstäker. Hierzu zählen Peroxo-Salze, Siliciumdioxid-Verbindungen sowie insbesondere kationisierte Heterocyclen.

Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Der Einsatz von Persulfatsalzen bzw. Peroxodisulfatsalzen erfolgt in der Regel in Form eines gegebenenfalls entstaubten Pulvers.

Die erfindungsgemäßen Zusammensetzungen können anstelle und/oder zusätzlich zu den festen Peroxo-Salzen einen weiteren Bleichkraftverstärker enthalten. Als Bleichkraftverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Leistung der Oxidationsmittelzubereitung kann der erfindungsgemäßen Zusammensetzung daher zusätzlich mindestens eine gegebenenfalls hydratisierte SiO₂-Verbindung zugesetzt werden. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Die gegebenenfalls hydratisierten SiO₂-Verbindungen können in verschiedenen Formen
vorliegen. Erfindungsgemäß bevorzugt werden die SiO₂-Verbindungen in Form von Kieselgelen (Silicagel) oder als Wasserglas eingesetzt. Erfindungsgemäß besonders bevorzugt sind Wassergläser. Erfindungsgemäß besonders bevorzugte Wassergläser werden unter den Bezeichnungen Ferrosil 119, Natronwasserglas 40/42, Portil A, Portil AW, Portil W und Britesil C20 vertrieben.

Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Die Aufhellmittel sind in dem Mittel bevorzugt in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether-und Amidgruppen sowie Hydroxylgruppen enthalten sein. Bevorzugte anionische Tenside sind Seifen, Alkylsulfate, Alkylethersulfate und Ethercarbonsäuren mit 8 bis 22 C-Atomen in der Alkylgruppe und bis zu 12 Glykolethergruppen im Molekül. Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet. Weiterhin hat es sich als vorteilhaft erwiesen, wenn die erfindungsgemäßen Färbe- oder Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als nichtionische Tenside eignen bevorzugt sich Alkylpolyglykoside, insbesondere C₈-C₂₂-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Erfindungsgemäß besonders bevorzugt sind gesättigte oder ungesättigte C₁₀ -C₂₂ Fettalkohole mit jeweils 2 bis 12 Mol Ethylenoxid pro Mol Fettalkohol (z.B. Laureth-2 oder Ceteareth-20). Zubereitungen mit hervorragenden Eigenschaften werden ebenfalls erhalten, wenn sie als nichtionische Tenside Fettsäureester von ethöxyliertem Glycerin enthalten. Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise Assoziativpolymere mit Fettalkylkette, kationische Polymere, nichtionische Polymere (Vinylpyrrolidinon/Vinylacrylat-Copolymere, Polyvinylpyrrolidinon, Vinylpyrrolidinon/VinylacetatCopolymere, Polyethylenglykole und Polysiloxane); zwitterionische und amphotere Polymere (Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (Phospholipide, wie Sojalecithin, Ei-Lecitin, Kephaline sowie Silikonöle); zusätzliche Proteinhydrolysate pflanzlicher oder tierischer Herkunft (Elastin-, Collagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, B₃, Bs, B₆, C, E, F und H; Pflanzenextrakte (aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl, Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol /

Acrylamid-Copolymere); Perlglanzmittel (Ethylenglykolmonostearat sowie PEG-3-distearat); Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Bevorzugt weisen die erfindungsgemäßen Mittel einen pH-Wert im Bereich von 4 bis 12 auf. Im Fall von Oxidationsfärbemitteln und Aufhellmitteln findet die Anwendung der Färbemittel vorzugsweise in einem alkalischen Milieu statt, bevorzugt bei einem pH-Wert im Bereich von 8,0 bis 12,0 und besonders bevorzugt von 9,0 bis 11,0. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen, ausgewählt aus 2-Aminoethan-1-ol (Monoethanolamin), 2-Amino-2-methylpropan-1-ol, 2-Amino-2-methyl-propan-1,3-diol, Monoisopropanolamin und Triethanolamin. Die basischen Aminosäuren werden bevorzugt ausgewählt aus Arginin und Lysin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Schließlich ist ein weiteres bevorzugtes Alkalisierungsmittel Ammoniak.

Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Im Falle eines oxidativen Färbemittels, bei dem die Oxidationsfarbstoffvorprodukte zunächst getrennt von einer Oxidationsmittelzubereitung, enthaltend bevorzugt Wasserstoffperoxid, vorliegen, ist dieses Vorgehen besonders bevorzugt.

Ein zweiter Gegenstand der vorliegenden Erfindung ist daher eine Mehrkomponenten Verpackungseinheit ("Kit-of-parts") zur Farbveränderung keratinischer Fasern wobei die Mehrkomponenten Verpackungseinheit einen ersten separat verpackten Container (A) enthält, der mindestens ein Mittel gemäß erstem Erfindungsgegenstand enthält, und mindestens einen zweiten separat verpackten Container (B) enthält, der mindestens ein Mittel enthaltend Wasserstoffperoxid, enthält.

Unter Container der Mehrkomponenten-Verpackungseinheit wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Weiterhin kann es erfindungsgemäß besonders vorteilhaft sein, wenn das genannte Kit-of-Parts mindestens ein weiteres Haarbehandlungsmittel in einem zusätzlichen Container enthält, insbesondere eine Konditioniermittelzubereitung oder ein Blondierpulver mit Peroxodisulfaten. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

In einer Ausführungsform des zweiten Erfindungsgegenstands enthält die Zubereitung des Containers (A) als farbverändernde Verbindung mindestens ein Oxidationsfarbstoffvorprodukt. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Die Mittel des ersten Erfindungsgegenstands können in Verfahren zur Farbveränderung menschlicher Haare genutzt werden. Dabei wird ein Mittel des ersten Erfindungsgegenstands auf das Haar aufgetragen und für einen Zeitraum von 3 bis 45 Minuten, bevorzugt 5 bis 30 Minuten im Haar belassen. Anschließend wird das Haar mit Wasser und/oder
einem handelsüblichen Shampoo gespült. Gewünschtenfalls kann das Haar anschließend mit einem weiteren Mittel behandelt werden und danach erneut mit Wasser und/oder einem handelsüblichen Shampoo gespült werden.

Ein weiterer Gegenstand der vorliegenden Erfindung sind daher ein Verfahren zur Farbveränderung keratinischer Fasern, wobei
a. gewünschtenfalls ein Vorbehandlungsmittel M1 auf die Faser aufgebracht wird, dann
b. ein Mittel zur Farb- oder Formveränderung M2 auf der Faser zur Anwendung kommt, wobei gewünschtenfalls dem Mittel M2 vor der Anwendung ein weiteres Mittel M3 zugegeben wird,
c. diese Mittel nach einer Zeit von 5-45 Minuten von der Faser abgespült werden
d. gewünschtenfalls ein weiteres Mittel M4 auf der Faser zur Anwendung kommt
e. und nach der Behandlung gegebenenfalls ein Nachbehandlungsmittel M5 auf die Faser aufgetragen und nach einer Einwirkzeit von 1 bis 10 Minuten wieder abgespült wird, wobei mindestens das Mittel M2 ein Mittel gemäß dem ersten Erfindungsgegenstand ist.

Die Auftragungs- und die Einwirktemperatur der Farbveränderungszubereitung beträgt Raumtemperatur bis 45°C. Gegebenenfalls kann die Wirkung der Farbveränderungszubereitung durch externe Wärmezufuhr, wie beispielsweise mittels einer Wärmehaube, verstärkt werden. Die bevorzugte Einwirkdauer der Farbveränderungszubereitung auf die keratinische Faser beträgt 3 bis 45 min, bevorzugt 5 bis 30 min. Nach Beendigung der Einwirkdauer wird das verbliebene Farbveränderungsmittel aus den keratinischen Fasern mit Hilfe einer Reinigungszubereitung oder Wasser ausgewaschen. Gegebenenfalls wird der Vorgang mit einem weiteren Mittel wiederholt. Nach dem Auswaschen werden die keratinischen Fasern gegebenenfalls mit einem Handtuch oder einem Heißluftgebläse getrocknet. Die Auftragung der Farbveränderungszubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Farbveränderungsmittel mit einer Applikationshilfe auf die keratinischen Fasern aufzutragen.

In einer besonderen Ausführungsform dieses Verfahrens wird das anwendungsbereite Farbveränderungsmittel durch Vermischen der getrennt verpackten Mittel der Mehrkomponenten-Vverpackungseinheit des zweiten Erfindungsgegenstands hergestellt, auf die keratinischen Fasern aufgetragen, für eine bestimmte Einwirkzeit im Haar belassen und das Haar anschließend mit Wasser und/oder einem handelsüblichen Shampoo ausgespült.

Bezüglich weiterer bevorzugter Ausführungsformen der erfindungsgemäßen Verfahren gilt mutatis mutandis das zu den erfindungsgemäßen Mitteln Gesagte.

### BEISPIELE

Die Mengenangaben verstehen sich, soweit nichts anderes vermerkt ist, jeweils in Gewichtsprozent.

### 1 Herstellung der Zubereitungen

### 1.1 Beispiel 1 - Blondiercremes

| Beschreibung | Zubereitung A | Zubereitung B |
|---|---|---|
| Wasser, vollentsalzt | AD 100 | AD 100 |
| Cetearylalkohol | 11 | 11 |
| Kokosalkylalkohol | 2,75 | 2,75 |
| Sodiumlaureth Sulfat | 7,2 | 7,2 |
| Ceteareth-20 | 0,25 | 0,25 |
| Ammoniumsulfat | 1 | 1 |
| Natriumsilikat 40/42 | 0,5 | 0,5 |
| HEDP 60 Gew.-% | 0,2 | 0,2 |
| Parfum | 0,4 | 0,4 |
| Ammoniak 25 % | 7,6 | 7,6 |
| Silsoft AX | | 4 |
| Gluadin W 40 | 0,35 | 0,35 |
| Vitamin F forte | 1 | 1 |

| | | |
|---|---|---|
| Eingesetzte Rohstoffe: Silsoft AX (INCI-Name: Bis-Cetearyl Amodimethicone [Momentive]) Gluadin W 40 (INCI-Name: Hydrolyzed Wheat Gluten/Hydrolyzed Wheat Protein (Solids 40-44%in H2_O) [Cognis]) | | |

**1.2 Beispiel 2 -Oxidationsmittelzubereitung**

| Beschreibung | Zubereitung C | Zubereitung D |
|---|---|---|
| Wasser, vollentsalzt | AD 100 | AD 100 |
| Sodiumlaureth Sulfat 27% | 0,6 | 0,6 |
| HEDP 60 Gew.-% | 1,5 | 1,5 |
| Ammoniak 25 % | 0,62 | 0,62 |
| Dipicolinsäure | 0,1 | 0,1 |
| Dinatriumpyrophosphat | 0,03 | 0,03 |
| Dow Corning DB 110 A | 0,067 | 0,067 |
| Acrylates Copolymer | 3,4 | 3,4 |
| Wasserstoffperoxid 50 % | 22,4 | 12 |

| | | |
|---|---|---|
| Erfindungsgemäße Mittel: Dow Corning DB 110 A (INCI-Name: non-ionic silicone emulsion (10% in H2O) [Dow Corning]) | | |

Die Blondiercremes gemäß Zubereitungen A und B wurden direkt vor der Anwendung je 1:1 mit der Oxidationsmittelzubereitung C zur anwendungsbereiten Blondiercreme vermischt.

### 1.3 Beispiel 3 - Färbecremes

| **Beschreibung** | **Zubereitung D** | **Zubereitung E** | **Zubereitung F** |
|---|---|---|---|
| Wasser, vollentsalzt | AD 100 | AD 100 | AD 100 |
| Cetearylalkohol | 8,1 | 8,1 | 8,1 |
| Kokosalkylalkohol | 2,8 | 2,8 | 2,8 |
| Sodiumlaureth Sulfat | 2,7 | 2,7 | 2,7 |
| Plantapon LGC | 5 | 5 | 5 |
| Ceteareth-12 | 0,25 | 0,25 | 0,25 |
| Ceteareth-20 | 0,25 | 0,25 | 0,25 |
| Isostearinsäure | 2 | 2 | 2 |
| Myristic Acid | 0,5 | 0,5 | 0,5 |
| Produkt W 37194 | | | 1 |
| Kaliumhydroxid 50% | 1,2 | 1,2 | 1,2 |
| 5-Amino-2-Methyl-Phenol | 0,5 | 0,5 | 0,5 |
| Propandiol-1,2 | 1,5 | 1,5 | 1,5 |
| 1-Hydroxy-4,5-Diamino-Pyrazolsulfat | 1,0 | 1,0 | 1,0 |
| Natriumsulfit | 0,5 | 0,5 | 0,5 |
| Ascorbinsäure | 0,4 | 0,4 | 0,4 |
| Ammoniumsulfat | 0,4 | 0,4 | 0,4 |
| Natriumsilikat 40/42 | 0,5 | 0,5 | 0,5 |
| HEDP 60 Gew.-% | 0,2 | 0,2 | 0,2 |
| Parfum | 0,4 | 0,4 | 0,4 |
| Ammoniak 25 % | 6,3 | 6,3 | 6,3 |
| PEG-12 Dimethicone | | 4 | |
| Silsoft AX | 4 | | |

### Eingesetzte Rohstoffe:

Plantapon® LGC (INCI-Name: Sodium lauryl glucose carboxylate (15-25%), Lauryl glucoside (10-20%), Water [Cognis])
Produkt W 37194 (INCI-Name: ACRYLAMIDOPROPYLTRIMONIUM CHLORIDE/ACRYLATES COPOLYMER (Solids 19-21% in H2O) [Bozzetto GmbH])
Silsoft AX (INCI-Name: Bis-Cetearyl Amodimethicone [Momentive])

Die Färbecremes gemäß Zubereitungen E, F und G wurden direkt vor der Anwendung je 1:1 mit der Oxidationsmittelzubereitung D anwendungsbereiten Färbecreme vermischt.

### 2. Durchführung des Tests zur Kämmbarkeit

Es wurden Haarsträhnen der Firma Kerling International, Typ: European natural hair 7/0 (lot# 04/2010, N71, 12 cm, Gewicht 1) verwendet. Die Strähnen wurden mit einer 3%igen AM Lösung von Texapon NSO in Wasser (pH 6-7) gewaschen und mindestens 24 Stunden bei 25°C und 25% relative Luftfeuchtigkeit gelagert.
4g der anwendungsbereiten Blondiercreme bzw. der anwendungsbereiten Färbecreme wurden pro Gramm Haar auf die Teststrähnen aufgetragen. Die Einwirkzeit für die Blondierungen betrug 45 Minuten bei 32°C für die Färbungen 30 Minuten bei 32°C Anschließend wurden die Strähnen für 2 Minuten mit Wasser bei 32°C gespült. Die Referenzsträhnen wurden kurz vor dem Kämmen 5 Minuten in Wasser getränkt.
Nach 3 Vorkämm-Strichen wurden die einzelnen Strähnen bei langsamer Rotation 10 Mal mit einem Kamm der Firma Hercules Sägemann (hard rubber comb, fine-toothed) gekämmt und die Arbeit der Nasskämmbarkeit gemessen.

### Ergebnisse

### Nasskämmbarkeit Blondierung

| Zubereitung | Aufgewendete Kraft in mJ |
|---|---|
| Referenz (unbehandelt) | 229 |
| Zubereitung A mit C (erfindungsgemäß) | 304 |
| Zubereitung B mit C (Vergleich) | 498 |

### Nasskämmbarkeit Haarfärbung

| Zubereitung | Aufgewendete Kraft in mJ |
|---|---|
| Referenz (unbehandelt) | 200 |
| Zubereitung E mit D (erfindungsgemäß) | 136 |
| Zubereitung F mit D (Vergleich 1) | 306 |
| Zubereitung G mit D (Vergleich 2) | 282 |

Die erfindungsgemäßen Mittel pflegen das behandelte Haar besser und erzielen eine deutlich bessere Nasskämmbarkeit als solche Mittel, die nicht erfindungsgemäß sind.

## Patentansprüche

1. Mittel zur Farbveränderung keratinischer Fasern enthaltend in einem kosmetisch geeigneten Träger
a. mindestens eine farbgebende Verbindung ausgewählt aus mindestens einem Oxidationsfarbstoffvorprodukt
**dadurch gekennzeichnet, dass** das Mittel weiterhin
b. mindestens eine Verbindung der Formel (I) enthält. 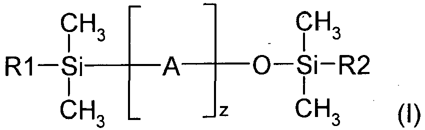 mit 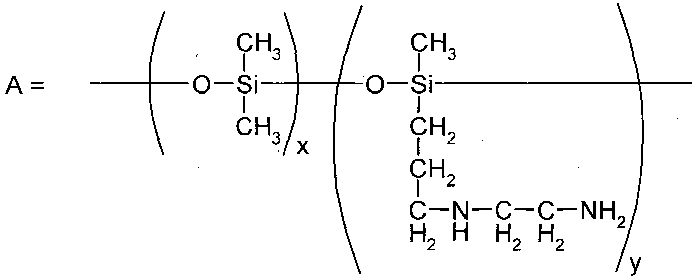 worin
i. x und y unabhängig voneinander für Werte zwischen 1 und 100 stehen und
ii. z für Werte zwischen 1 und 100 steht, wobei, falls z ≥ 2, die jeweiligen Werte x und y in einem Strukturelement A jeweils unabhängig von vorangehenden Strukturelementen A gewählt werden können
und
iii. R1 und R2 unabhängig voneinander für lineare C₁₄-C₂₀-Alkylketten stehen.

2. Mittel nach Anspruch 1 **dadurch gekennzeichnet, dass** mindestens eine Verbindung der Formel (I) Bis-Cetearyl Amodimethicon ist.

3. Mittel nach einem der Ansprüche 1 bis 2, **dadurch gekennzeichnet, dass** es bezogen auf sein Gewicht 0,005 bis 5 Gew.-%, bevorzugt, 0,1 bis 4,5 Gew.-%, besonders bevorzugt 1 bis 3 Gew.-% und insbesondere bevorzugt 1,5 bis 2,5 Gew.-% der Verbindung/en der Formel (I) enthält.

4. Mittel nach einem der Ansprüche 1 bis 3, **dadurch gekennzeichnet, dass** es bezogen auf sein Gewicht 0,001 bis 10 Gew.-%, bevorzugt, 0,01 bis 8 Gew.-%, besonders bevorzugt 0,1 bis 5 Gew.-% und insbesondere bevorzugt 0,5 bis 3 Gew.-% mindestens eines Oxidationsfarbstoffvorproduktes enthält.

5. Mittel nach einem der Ansprüche 1 bis 4, **dadurch gekennzeichnet, dass** es zusätzlich ein Oxidationsmittel, ausgewählt aus Wasserstoffperoxid sowie seinen festen Anlagerungsprodukten an organische und anorganische Verbindungen, enthält.

6. Mittel nach einem der Ansprüche 1 bis 5, **dadurch gekennzeichnet, dass** es als Oxidationsmittel bezogen auf sein Gewicht mindestens 1 Gew.-% Wasserstoffperoxid enthält.

7. Mehrkomponenten Verpackungseinheit zur Farbveränderung keratinischer Fasern, **dadurch gekennzeichnet, dass** es einen ersten separat verpackten Container (A) enthält, der ein Mittel nach einem der Ansprüche 1 bis 4 enthält, und mindestens einen zweiten separat verpackten Container (B) enthält, der ein Mittel enthaltend Wasserstoffperoxid enthält.

8. Verfahren zur Farbveränderung keratinischer Fasern bei dem
a. gewünschtenfalls ein Vorbehandlungsmittel M1 auf die Faser aufgebracht wird, dann
b. ein Mittel zur Farbveränderung M2 auf der Faser zur Anwendung kommt wobei gewünschtenfalls dem Mittel M2 vor der Anwendung ein weiteres Mittel M3 zugegeben wird,
c. diese Mittel nach einer Zeit von 5 bis 45 Minuten von der Faser abgespült wird
d. gewünschtenfalls ein weiteres Mittel M4 auf der Faser zur Anwendung kommt
e. und nach der Behandlung gegebenenfalls ein Nachbehandlungsmittel M5 auf die Faser aufgetragen und nach einer Einwirkzeit von 1 bis 10 Minuten wieder abgespült wird,
**dadurch gekennzeichnet, dass** mindestens das Mittel M2 ein Mittel gemäß Anspruch 1 ist.

## Claims

1. An agent for changing the color of keratin fibers, containing in a cosmetically suitable carrier
a. at least one color-imparting compound selected from at least one oxidation dye precursor,
**characterized in that** the agent additionally contains
b. at least one compound of formula (I) 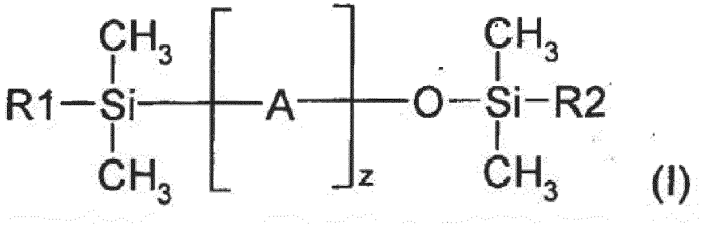 where 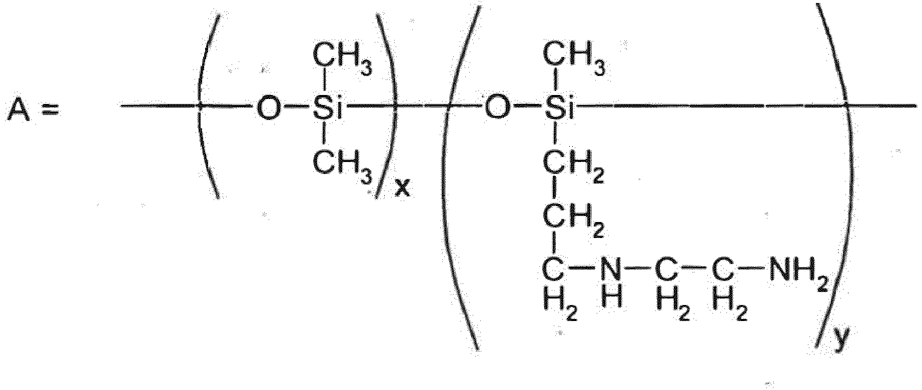 in which
i. x and y represent, independently of one another, values between 1 and 100 and
ii. z represents values between 1 and 100, where, if z is ≥ 2, the respective values x and y in a structural element A can be selected in each case independently of preceding structural elements A and
iii. R1 and R2 represent, independently of one another, linear C₁₄-C₂₀ alkyl chains.

2. The agent according to claim 1, **characterized in that** at least one compound of formula (I) is Bis-Cetearyl Amodimethicone.

3. The agent according to one of claims 1 to 2, **characterized in that** it contains, based on its weight, 0.005 to 5 wt.%, preferably 0.1 to 4.5 wt.%, particularly preferably 1 to 3 wt.% and more particularly preferably 1.5 to 2.5 wt.%, of the compound(s) of formula (I).

4. The agent according to one of claims 1 to 3, **characterized in that** it contains, based on its weight, 0.001 to 10 wt.%, preferably 0.01 to 8 wt.%, particularly preferably 0.1 to 5 wt.% and more particularly preferably 0.5 to 3 wt.%, of at least one oxidation dye precursor.

5. The agent according to one of claims 1 to 4, **characterized in that** it additionally contains an oxidizing agent, selected from hydrogen peroxide and its solid addition products with organic and inorganic compounds.

6. The agent according to one of claims 1 to 5, **characterized in that** it contains, based on its weight, at least 1 wt.% hydrogen peroxide as the oxidizing agent.

7. A multicomponent packaging unit for changing the color of keratin fibers, **characterized in that** it contains a first separately packaged container (A) which contains an agent according to one of claims 1 to 4, and at least one second separately packaged container (B) which contains an agent containing hydrogen peroxide.

8. A method for changing the color of keratin fibers, in which
a. a pretreatment agent M1 is applied to the fiber if desired, then
b. a color-changing agent M2 is applied to the fiber, an additional agent M3 being added, if desired, to the agent M2 before said agent is applied,
c. said agent is rinsed off the fiber after a time of from 5 to 45 minutes,
d. if desired, an additional agent M4 is applied to the fiber,
e. and a post-treatment agent M5 is optionally applied to the fiber after treatment and is rinsed off again after a contact time of from 1 to 10 minutes,
**characterized in that** at least the agent M2 is an agent according to claim 1.

## Revendications

1. Agent pour la modification de couleur de fibres kératiniques contenant, dans un support approprié du point de vue cosmétique,
a. au moins un composé colorant choisi parmi au moins un précurseur de colorant d'oxydation
**caractérisé en ce que** l'agent contient en outre
b. au moins un composé de formule (I). 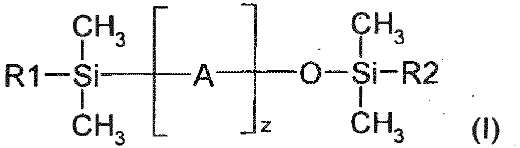 avec 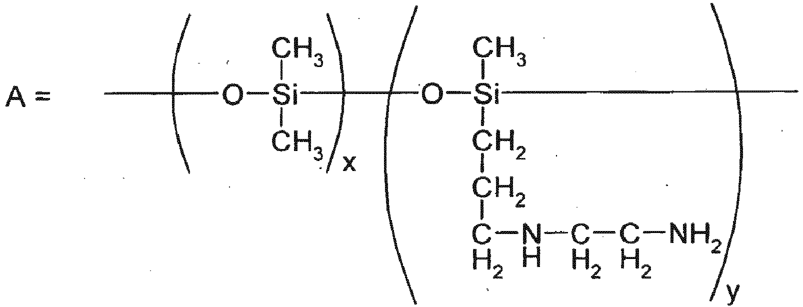 dans laquelle
i. x et y représentent indépendamment l'un de l'autre des valeurs comprises entre 1 et 100 et
ii. z représente des valeurs comprises entre 1 et 100, si z ≥ 2, les valeurs respectives de x et y dans un élément structurel A pouvant être choisies indépendamment des éléments structurels A précédents
et
iii. R1 et R2 représentent indépendamment l'un de l'autre des chaînes alkyle linéaires en C₁₄ à C₂₀.

2. Agent selon la revendication 1, **caractérisé en ce qu'**au moins un composé de formule (I) est le bis-cétéaryl amodiméthicone.

3. Agent selon l'une des revendications 1 et 2, **caractérisé en ce qu'**il contient, rapporté à son poids, 0,005 à 5 % en poids, de préférence 0,1 à 4,5 % en poids, de manière particulièrement préférée 1 à 3 % en poids et de manière tout particulièrement préférée 1,5 à 2,5 % en poids du ou des composés de formule (I).

4. Agent selon l'une des revendications 1 à 3, **caractérisé en ce qu'**il contient, rapporté à son poids, 0,001 à 10 % en poids, de préférence 0,01 à 8 % en poids, de manière particulièrement préférée 0,1 à 5 % en poids et de manière tout particulièrement préférée 0,5 à 3 % en poids d'au moins un précurseur de colorant d'oxydation.

5. Agent selon l'une des revendications 1 à 4, **caractérisé en ce qu'**il contient en plus un agent d'oxydation, choisi parmi le peroxyde d'hydrogène ainsi que ses produits d'addition solides sur des composés organiques et inorganiques.

6. Agent selon l'une des revendications 1 à 5, **caractérisé en ce qu'**il contient comme agent d'oxydation, rapporté à son poids, au moins 1 % en poids de peroxyde d'hydrogène.

7. Unité de conditionnement multi-composants pour la modification de couleur de fibres kératiniques, **caractérisée en ce qu'**il contient un premier réservoir (A) conditionné séparément et qui contient un agent selon l'une des revendications 1 à 4, et au moins un deuxième réservoir (B) conditionné séparément et qui contient un agent contenant du peroxyde d'hydrogène.

8. Procédé pour la modification de couleur de fibres kératiniques dans lequel
a. un agent de prétraitement M1 est, éventuellement, appliqué sur les fibres, puis
b. un agent de modification de couleur M2 est appliqué sur les fibres, un autre agent M3 étant, éventuellement, ajouté à l'agent M2 avant l'application,
c. ces agents étant retirés des fibres par rinçage après un temps de 5 à 45 minutes
d. un autre agent M4 étant éventuellement appliqué sur les fibres
e. et après le traitement, un agent de post-traitement M5 étant éventuellement appliqué sur les fibres et rincé au bout d'un temps d'action de 1 à 10 minutes,
**caractérisé en ce qu'**au moins l'agent M2 est un agent selon la revendication 1.
